# FASCICULE DE BREVET EUROPEEN

(11) **EP 3 956 628 B1**
(45) Date de publication et mention de la délivrance du brevet: **25.09.2024**
(21) Numéro de dépôt: 20717231.3
(22) Date de dépôt: 15.04.2020
(51) Int. Cl.: F42B 33/02, G01G 13/02

(54) **PROCÉDÉ OPTIMISÉ DE REMPLISSAGE D'UNE CHARGE PYROTECHNIQUE ET SYSTÈME METTANT EN OEUVRE UN TEL PROCÉDÉ**
OPTIMIERTES VERFAHREN ZUM FÜLLEN EINER PYROTECHNISCHEN LADUNG UND SYSTEM ZUR DURCHFÜHRUNG EINES SOLCHEN VERFAHRENS
OPTIMISED METHOD FOR FILLING A PYROTECHNIC CHARGE AND SYSTEM IMPLEMENTING SUCH A METHOD

(30) Priorité: 15.04.2019 FR 1904010
(43) Date de publication de la demande: 23.02.2022
(73) Titulaire: Crossject, 21000 Dijon (FR)
(72) Inventeur: AURIEL, Christophe, 21270 Binges (FR); MONNIER, Stéphane, 21000 Dijon (FR); CAVILLON, Michel, 21000 Dijon (FR); RESSOUCHE, Maxime, 21000 Dijon (FR)
(74) Mandataire: Germain Maureau
(86) Numéro de dépôt international: PCT/EP2020/060591
(87) Numéro de publication internationale: WO 2020/212422

(56) Documents cités:
- DE-A1- 4 405 253
- SE-B- 420 949
- US-A- 6 121 556

## Description

L'invention se rapporte à un procédé de remplissage d'une charge pyrotechnique. L'invention se rapporte également à un système de remplissage pouvant mettre en oeuvre ledit procédé.

Le procédé et le système de remplissage selon l'invention trouve une application dans le domaine général du remplissage d'une charge pyrotechnique.

Le procédé et le système de remplissage selon l'invention trouve une application particulière lorsqu'ils sont appliqués à un dispositif d'injection sans aiguille comprenant une telle charge pyrotechnique.

On connaît des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif à usage thérapeutique en médecine humaine ou vétérinaire.

Le principe actif est constitué par un liquide pouvant être visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié.

Un tel dispositif d'injection comporte de manière connue, par exemple dans la demande de brevet FR 2 815 544 A, un corps comprenant successivement un générateur de gaz, une chambre d'expansion, un réservoir contenant le principe actif et un système d'injection.

Un tel générateur à gaz comporte un contenant recevant un mélange de poudre bi-composition formé par une poudre vive et par une poudre lente.

Le réservoir est inséré dans un logement tubulaire du corps du dispositif d'injection, en étant obturé par un piston supérieur, ou amont, et un piston inférieur, ou aval. L'extrémité libre inférieure du réservoir coopère avec le système d'injection comprenant une buse d'injection comportant plusieurs canaux d'injection s'étendant axialement suivant un axe d'injection. Le diamètre des canaux d'injection est compatible avec la granulométrie du principe actif pour éviter qu'il soit obturé.

Pour permettre l'injection du principe actif, le corps est monté coulissant dans un capot creux enveloppant le corps, de bas en haut suivant un axe de coulissement, entre une position de repos et une position d'injection, l'entraînement du corps étant réalisé lorsque l'utilisateur appui la buse d'injection sur sa peau. Le déplacement du corps dans le capot permet le déclenchement du générateur de gaz, générant un gaz sous pression qui entraîne en déplacement les pistons pour injecter le principe actif à travers la peau du patient, en passant par la buse d'injection.

Dans cette position d'injection, le principe actif est éjecté depuis les canaux de la buse d'injection, dans une direction d'injection, selon des jets ayant une pression d'injection prédéterminée permettant au principe actif de traverser la peau du patient à la profondeur souhaitée pour assurer une injection optimale de celui-ci. La pression d'injection de ces jets est donc déterminante de la profondeur d'injection dans la peau du principe actif injecté.

Pour permettre une injection réussie, les jets en sortie de la buse d'injection doivent être délivrés selon cette pression d'injection pendant un temps d'injection prédéterminé garantissant l'injection de la quantité de principe actif désirée.

Ces grandeurs de pression ou alternativement de force d'injection et de temps d'injection sont, bien entendu, dépendantes du principe actif à injecter.

Ainsi, chacune des poudres de la charge pyrotechnique doit être dosée avec précision dans chaque contenant. La précision de dosage de la poudre vive peut, par exemple, être de plus ou moins 1 mg, alors que celle de la poudre lente peut être de plus ou moins 2 mg.

Lorsqu'il est souhaité maintenir une cadence optimale pour la production en série de charges pyrotechniques, il est constaté que la poudre d'un certain nombre de charges pyrotechniques est incorrectement dosée, conduisant alors à une non-conformité des dispositifs d'injection sur lesquels ces charges pyrotechniques sont montées.

Par conséquent, il est souvent retenu de réduire la cadence de production pour doser précisément les charges pyrotechniques, limitant alors la production de lots de charge pyrotechnique en acceptant toutefois un nombre réduit de charges pyrotechniques non conforme.

Le document SE 420 949 B décrit un procédé de remplissage d'un contenant d'une charge pyrotechnique à l'aide d'un système de remplissage. Le document DE 44 05 253 A1 décrit un dispositif de régulation de débit pour matériau.

Il existe donc un besoin pour un procédé optimisé de remplissage d'une charge pyrotechnique permettant de doser précisément une telle charge pyrotechnique tout en assurant une cadence de production optimale.

A cet effet, l'invention a pour objet un procédé de remplissage selon la revendication 1 et un système de remplissage selon la revendication 9.

Grâce à un tel procédé de remplissage, il est possible d'assurer une distribution constante et précise de la poudre dans la cuvette du dispositif de mesure.

On entend par taux de remplissage du contenant de la charge pyrotechnique, le rapport entre la quantité de poudre mesurée dans la cuvette et la quantité de poudre désirée dans le contenant de la charge pyrotechnique.

La quantité de poudre désirée correspond à une valeur de consigne prédéterminée de poids, plus ou moins une tolérance prédéterminée.

Par exemple, la quantité de poudre désirée dans le contenant de la charge pyrotechnique peut varier de 8mg à 60mg.

Par exemple, la tolérance prédéterminée est comprise entre 0,25mg et 2mg. Par exemple, la tolérance prédéterminée est de 0,5mg. Dans un autre exemple, la tolérance prédéterminée est de 1mg.

La cuvette peut alors avantageusement permettre de préparer la quantité de poudre désirée dans la charge pyrotechnique pendant que le contenant est acheminé sur une ligne de production.

Autrement dit, un tel procédé permet de préparer la quantité de poudre désirée devant être versée dans le contenant indépendamment de celui-ci. Ainsi, lorsque la quantité de poudre mesurée est supérieure à une valeur seuil prédéterminée, correspondant à la quantité de poudre désirée dans la charge pyrotechnique, la poudre incorrectement dosée peut être récupérée sans nécessiter d'identifier le contenant comme non conforme. En effet, la poudre incorrectement dosée peut ainsi être récupérée et peut être réintroduite dans le réservoir de distribution.

Selon un aspect, la taille maximale des grains de la poudre est calibrée par un tamis placé en amont du dispositif d'acheminement.

En effet, lorsqu'un ordre d'arrêter l'acheminement de la poudre dans la cuvette du dispositif de mesure est donné, un certain temps de latence s'écoule avant qu'un dernier grain ne tombe dans la cuvette. Ceci est dû à la fois au temps de réponse d'une chaîne d'acquisition d'information reçue par une unité de contrôle du système de remplissage et à l'inertie des grains. Ce temps de latence génère une incertitude quant à la quantité de poudre acheminée dans la cuvette. Le tamis disposé en amont du dispositif d'acheminement permet de de limiter cette incertitude.

Selon une variante de réalisation du procédé de l'invention, la séquence de commande en fréquence de vibration peut comprendre une première phase dans laquelle le dispositif d'acheminement de poudre est mis en vibration selon une première fréquence jusqu'à atteindre un premier taux de remplissage du contenant.

Par exemple, le premier taux de remplissage du contenant est compris entre 50% et 80%, préférentiellement au moins égal à 70%.

Selon un aspect, la séquence de commande en fréquence de vibration peut comprendre une deuxième phase, postérieure à la première phase, dans laquelle le dispositif d'acheminement de poudre est mis en vibration selon une deuxième fréquence jusqu'à atteindre un deuxième taux de remplissage du contenant.

Par exemple, le deuxième taux de remplissage du contenant est supérieur à 80%. Par exemple, le deuxième taux de remplissage du contenant est supérieur à 90 %.

Selon un aspect, la première fréquence est supérieure à la deuxième fréquence.

Par exemple, la première fréquence est comprise entre 16Hz et 18Hz. Par exemple, la première fréquence est sensiblement égale à 17hz.

Par exemple, la deuxième fréquence est comprise entre 13Hz et 16Hz. Par exemple, la deuxième fréquence est sensiblement égale à 15hz.

Selon un aspect, la séquence de commande en fréquence de vibration peut comprendre une troisième phase, dite « de stabilisation » dans laquelle la mise en vibration du dispositif d'acheminement de poudre est stoppée jusqu'à la stabilisation de la quantité de poudre mesurée. Par exemple, la troisième phase de stabilisation a une durée comprise entre 1000 ms et 3000ms. Par exemple, la troisième phase de stabilisation a une durée sensiblement égale à 2000ms.

Si la quantité de poudre mesurée, une fois stabilisée, correspond à la quantité de poudre désirée ou est supérieure à la quantité de poudre désirée, la séquence de commande en fréquence de vibration du dispositif d'acheminement est terminée.

Si la quantité de poudre mesurée, une fois stabilisée, est inférieure à la quantité de poudre désirée successivement à la troisième phase, il peut être prévu une quatrième phase comprenant la mise en vibration du dispositif d'acheminement de poudre selon une troisième fréquence pendant une durée prédéterminée.

Par exemple, la quatrième phase est mise en oeuvre lorsque le taux de remplissage est compris entre 95 et 98%.

On comprend que la quatrième phase est mise en oeuvre lorsque, après la troisième phase, la quantité de poudre mesurée est inférieure à la valeur de consigne prédéterminée moins la tolérance prédéterminée.

Par exemple, la durée prédéterminée de la quatrième phase est comprise entre 500ms et 1500ms. Par exemple, la durée prédéterminée de la quatrième phase est sensiblement égale à 1000ms.

Par exemple, la troisième fréquence est comprise entre 10Hz et 20Hz. Par exemple, la troisième fréquence est sensiblement égale à 14hz.

Selon un aspect, entre la deuxième phase et la troisième phase, la séquence de commande en fréquence de vibration peut comprendre une deuxième phase analogue dans laquelle le dispositif d'acheminement de poudre est mis en vibration selon une deuxième fréquence analogue jusqu'à atteindre un deuxième taux de remplissage analogue du contenant.

Par exemple, le deuxième taux de remplissage analogue est supérieur à 95%.

Par exemple, la deuxième fréquence analogue est inférieure à la deuxième fréquence. Par exemple, la deuxième fréquence analogue est comprise entre 8Hz et 13Hz. Par exemple, la deuxième fréquence analogue est sensiblement égale à 10Hz.

Selon un aspect, postérieurement à la quatrième phase, la séquence de commande en fréquence de vibration peut comprendre une cinquième phase de stabilisation, dans laquelle la mise en vibration du dispositif d'acheminement de poudre est stoppée jusqu'à la stabilisation de la quantité de poudre mesurée.

Par exemple, la cinquième phase de stabilisation a une durée comprise entre 1000 ms et 3000ms. Par exemple, la troisième phase de stabilisation a une durée d'environ 2000ms.

Si la quantité de poudre mesurée, une fois stabilisée, correspond à la quantité de poudre désirée ou est supérieure à la quantité de poudre désirée, la séquence de commande en fréquence de vibration du dispositif d'acheminement est terminée.

Si la quantité de poudre mesurée, une fois stabilisée, est inférieure à la quantité de poudre désirée, la quatrième phase et la cinquième phase sont répétées jusqu'à ce que la quantité de poudre mesurée, une fois stabilisée, corresponde à la quantité de poudre désirée.

Selon un aspect, préalablement à la première phase, la séquence de commande en fréquence de vibration peut comprendre une phase de démarrage, dans laquelle le dispositif d'acheminement de poudre est mis en vibration selon une fréquence de démarrage, pendant une durée de démarrage prédéterminée. Par exemple, la fréquence de démarrage est supérieure à la première fréquence.

Par exemple, la fréquence de démarrage est supérieure à 18Hz. Par exemple, la fréquence de démarrage est comprise entre 18Hz et 20Hz. Par exemple, la fréquence de démarrage est sensiblement égale à 19Hz.

Par exemple, la durée de démarrage prédéterminée est comprise entre 150ms et 250ms. Par exemple, la durée de démarrage prédéterminée est comprise entre 170ms et 210ms. Par exemple, la durée de démarrage prédéterminée est sensiblement égale à 190ms.

Selon une variante de l'invention, le changement de phase dans la mise en vibration du dispositif d'acheminement de poudre est réalisée en basculant directement de la fréquence de démarrage à la première fréquence et/ou de la première fréquence à la deuxième fréquence et/ou de la deuxième fréquence à la deuxième fréquence analogue et/ou de la deuxième fréquence analogue à une fréquence nulle et/ou d'une fréquence nulle à la troisième fréquence, et/ou de la troisième fréquence à une fréquence nulle.

Autrement dit, une courbe de la fréquence en fonction du taux de remplissage ou du temps, représentant les changements de la séquence de commande en fréquence de vibration peut être en forme d'escalier.

Autrement dit, lors d'un changement de phase, la fréquence passe d'une valeur à une autre valeur sans passer par des valeurs de fréquence intermédiaires.

Par exemple, lors du passage de la première phase à la deuxième phase, la fréquence de vibration du dispositif d'acheminement passe de la première fréquence à la deuxième fréquence, sans passer par des valeurs de fréquence intermédiaires.

Cela permet avantageusement d'atteindre la quantité de poudre désirée plus rapidement.

Selon une variante de l'invention, le changement de phase dans la mise en vibration du dispositif d'acheminement de poudre est réalisée en basculant linéairement de la fréquence de démarrage à la première fréquence et/ou de la première fréquence à la deuxième fréquence et/ou de la deuxième fréquence à la deuxième fréquence analogue et/ou de la deuxième fréquence analogue à une fréquence nulle et/ou d'une fréquence nulle à la troisième fréquence, et/ou de la troisième fréquence à une fréquence nulle.

Autrement dit, une courbe de la fréquence en fonction du taux de remplissage ou du temps, représentant les changements de la séquence de commande en fréquence de vibration peut comprendre, entre les paliers, des portions de courbe inclinées.

Autrement dit, lors d'un changement de phase, la fréquence passe d'une valeur à une autre valeur progressivement, c'est-à-dire en passant par des valeurs intermédiaires. Par exemple, lors du passage de la première phase à la deuxième phase, la fréquence de vibration du dispositif d'acheminement passe de la première fréquence à la deuxième fréquence, en passant par des valeurs de fréquence intermédiaires.

Cela permet avantageusement d'atteindre la quantité de poudre désirée de manière plus précise.

La séquence de commande selon l'une des variantes ci-dessus, ainsi que les première, deuxième, troisième fréquences et/ou la fréquence de démarrage et/ou deuxième fréquence analogue sont avantageusement choisies préalablement à la mise en vibration du dispositif d'acheminement, c'est-à-dire hors production.

La séquence de commande en fréquence de vibration permet d'assurer un remplissage initialement rapide du contenant de la charge pyrotechnique, puis progressivement diminué jusqu'à atteindre la quantité de poudre désirée. La cadence d'une ligne de production mettant en oeuvre une séquence de commande en fréquence de vibration telle que décrite ci-dessus s'en trouve augmentée.

Selon un aspect, le système de remplissage comprend en outre un dispositif de sélection, le dispositif de sélection comportant un bac récupérateur et un entonnoir inférieur.

Selon un aspect, le bac récupérateur est configuré pour récupérer la poudre de la cuvette lorsque celle-ci est incorrectement dosée. Par exemple, le bac récupérateur est configuré pour récupérer la poudre disposée dans la cuvette lorsque la quantité de poudre mesurée dépasse la quantité de poudre désirée.

Par exemple, le bac récupérateur est configuré pour récupérer la poudre disposée dans la cuvette lorsque la quantité de poudre mesurée dépasse la valeur de consigne prédéterminée plus la tolérance prédéterminée.

Selon un aspect, l'entonnoir inférieur est configuré pour permettre l'introduction de la poudre disposée dans la cuvette dans le contenant, lorsque la quantité de poudre mesurée contenue dans la cuvette correspond à la quantité de poudre désirée.

Par exemple, la cuvette est configurée pour réaliser une rotation d'environ 180° pour verser la poudre dans le bac récupérateur ou dans l'entonnoir inférieur.

Selon un aspect, ledit procédé comprend :
- une étape de mesure de quantité de poudre contenue la cuvette,
- lorsque la quantité de poudre contenue dans la cuvette est égale à une valeur prédéterminée correspondant à la quantité de poudre désirée, une étape de vérification de la position du dispositif de sélection dans laquelle il est vérifié que la cuvette est disposée en regard de l'entonnoir inférieur.

Grâce à ces étapes de procédé, seulement lorsque la quantité de poudre contenue dans la cuvette correspond à la quantité de poudre désirée, l'entonnoir inférieur récupère la poudre afin de l'introduire dans le contenant. Ainsi, le nombre de charges pyrotechniques considérées comme non conformes à la sortie de la chaîne de production est réduit.

Selon un aspect, si la cuvette n'est pas positionnée en regard de l'entonnoir inférieur, le procédé comprend une étape d'acheminement de l'entonnoir inférieur en regard de la cuvette.

Selon une autre variante de réalisation du procédé, le procédé comprend :
- lorsque la quantité de poudre contenue dans la cuvette n'est pas égale à la valeur prédéterminée correspondant à la quantité de poudre désirée, une étape de vérification de la position du dispositif de sélection dans laquelle il est vérifié que la cuvette est disposée en regard du bac récupérateur.

Grâce à cette étape de procédé, il est possible de récupérer le contenu de la cuvette incorrectement dosé, ce qui permet de réutiliser cette poudre.

Selon un aspect, si la cuvette n'est pas positionnée en regard du bac récupérateur, le procédé comprend une étape d'acheminement du bac récupérateur en regard de la cuvette.

Selon une autre variante de réalisation du procédé, le système de remplissage comprend ledit entonnoir inférieur, dit premier entonnoir inférieur, et un deuxième entonnoir inférieur pour la récupération de la poudre versée dans le premier entonnoir. Le deuxième entonnoir inférieur est configuré pour être accouplé au contenant de façon à pouvoir introduire la poudre dans le contenant en limitant les pertes.

Selon un aspect, le procédé comprenant :
- une étape d'acheminement du contenant de la charge pyrotechnique en regard du deuxième entonnoir inférieur,
- une étape d'accouplement du deuxième entonnoir inférieur avec le contenant de la charge pyrotechnique.

On comprend que l'accouplement du deuxième entonnoir inférieur avec le contenant est réalisé lorsqu'il a été déterminé que la cuvette contenait une quantité de poudre correspondant à la quantité de poudre désirée.

On comprend que le premier entonnoir inférieur est disposé en regard du deuxième entonnoir inférieur lorsqu'il a été déterminé que la cuvette contenait une quantité de poudre correspondant à la quantité de poudre désirée.

Grâce à ces étapes du procédé, il est possible de réaliser l'accouplement du contenant avec le deuxième entonnoir inférieur qu'une fois qu'il a été déterminé que la cuvette contenait une quantité de poudre correspondant à la quantité de poudre désirée. Selon un aspect, le système de remplissage comprend un entonnoir supérieur pour la récupération de la poudre contenue dans la cuvette. L'entonnoir supérieur est configuré pour être disposé sous la cuvette.

Selon un aspect, lorsque la quantité de poudre contenue dans la cuvette correspond à la quantité de poudre désirée, le premier entonnoir inférieur est disposé sous l'entonnoir supérieur.

Selon un aspect, lorsque la quantité de poudre contenue dans la cuvette ne correspond pas à la quantité de poudre désirée, le bac récupérateur est disposé sous l'entonnoir supérieur.

Selon un autre mode de réalisation de l'invention, le système de remplissage est rendu équipotentiel pour éviter la retenue de grains de poudre par phénomène d'électrostatisme.

Selon un autre mode de réalisation de l'invention, le procédé comprend une étape de mise en vibration dans laquelle au moins l'un des entonnoirs est mis en vibration constante.

Par exemple, au moins un des entonnoirs est mis en vibration à l'aide d'au moins un actionneur de vibrations tel qu'un actionneur piézo-électrique.

Avantageusement, chaque entonnoir est mis en vibration.

Selon une variante de réalisation du procédé, l'au moins un réservoir de distribution d'une poudre pyrotechnique est associé à deux dispositifs de mesure de poudre, chaque dispositif de mesure de poudre étant associé à un dispositif d'acheminement de poudre depuis le réservoir jusqu'à une cuvette du dispositif de mesure correspondant l'étape de régulation du débit de poudre étant mise en oeuvre lorsque la poudre est acheminée par l'un et/ou l'autre des dispositifs d'acheminement.

Ainsi, la cadence de production de charges pyrotechniques est augmentée.

Selon un aspect, le système de remplissage comprend pour chacune des cuvettes, un entonnoir supérieur de récupération de la poudre disposé en regard de la cuvette correspondante.

Selon un aspect, ledit procédé comprend :
- une étape de mesure de la quantité de poudre contenue dans chaque cuvette des dispositifs de mesure,
- lorsque la quantité de la poudre contenue dans au moins une cuvette correspond à la quantité de poudre désirée, une étape de vérification de la position du dispositif de sélection dans laquelle il est vérifié que la cuvette contenant la quantité de poudre correspondant à la quantité de poudre désirée est disposée en regard de l'entonnoir inférieur.

Ainsi, en plus des avantages mentionnés ci-dessus, la cadence de production n'est pas affectée si la quantité de poudre contenue dans l'une des cuvettes ne correspond pas à la quantité de poudre désirée.

On comprend que lorsque chaque cuvette est correctement dosée, l'une quelconque de ces cuvettes est retenue pour verser la poudre qu'elle contient dans le contenant de la charge pyrotechnique.

Selon un aspect, le procédé décrit ci-dessus est configuré pour être mis en oeuvre par un premier système de remplissage configuré pour remplir le contenant d'un premier type de poudre, et un deuxième système de remplissage configuré pour remplir le contenant d'un deuxième type de poudre.

Par exemple, le premier type de poudre est de la poudre lente et le deuxième type de poudre est de la poudre vive, ou inversement.

Selon un aspect, le contenant est configuré pour être acheminé vers le premier système de remplissage de sorte à être rempli du premier type de poudre, puis pour être acheminé vers le deuxième système de remplissage de sorte à être rempli du deuxième type de poudre.

Avantageusement, la charge pyrotechnique est prévue pour un dispositif d'injection sans aiguille dont un principe actif contenu dans un réservoir du dispositif d'injection peut avantageusement être choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Méthylnaltrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medroxyprogesterone acetate,
- Medroparine calcium,
- Methylprednisolone acetate
- Heparin calcium,
- Terbutaline.

La quantité de poudre est avantageusement choisie pour permettre une injection réussie, les jets en sortie d'une buse d'injection doivent être délivrés selon une pression d'injection pendant un temps d'injection prédéterminé garantissant l'injection de la quantité de principe actif désirée.

Le présent exposé concerne en outre un système de remplissage d'un contenant d'une charge pyrotechnique, le système de remplissage comprenant au moins :
- un réservoir de distribution d'une poudre pyrotechnique,
- un dispositif de mesure de cette poudre, et
- un dispositif d'acheminement de cette poudre depuis le réservoir jusqu'à une cuvette du dispositif de mesure.

Le système de remplissage comprend au moins un actionneur de vibrations, tel qu'un actionneur piézo-électrique, couplé au dispositif d'acheminement de cette poudre. De façon non limitative, l'actionneur de vibrations peut être de type électrique, mécanique ou pneumatique.

Selon un aspect, le système de remplissage est apte à mettre en oeuvre le procédé tel que défini ci-dessus.

Selon un aspect, le système de remplissage comprend en outre une unité de contrôle électronique configurée pour commander l'actionneur de vibration de sorte à mettre en oeuvre une séquence de commande en fréquence de vibration, la fréquence de vibration étant inversement proportionnelle au taux de remplissage du contenant de la charge pyrotechnique.

La séquence de commande de fréquence en vibration est conforme à l'une quelconque des caractéristiques susmentionnées.

Avantageusement, le dispositif de mesure est directement relié mécaniquement à un référentiel neutre, tel que le sol. Ainsi, il est possible de s'assurer que la mesure de la quantité de poudre contenue dans la cuvette ne soit pas dégradée par des perturbations mécaniques des éléments constituant le système de remplissage.

On comprendra que le système de remplissage comprend un châssis principal, ce châssis principal étant relié au référentiel neutre distinctement d'un châssis du dispositif de mesure de sorte que le dispositif de mesure est mécaniquement isolé des autres éléments constitutifs du système de remplissage.

Encore plus avantageusement, le dispositif de mesure est relié au référentiel neutre par une liaison souple anti vibration. Une telle liaison souple peut être réalisée par un ou plusieurs amortisseurs de vibrations.

Une telle configuration du système de remplissage permet d'améliorer la précision du dispositif de mesure dont les perturbations mécaniques des éléments constituant le système de remplissage n'influent pas sur le dispositif de mesure.

Avantageusement, l'au moins un actionneur de vibrations est directement relié mécaniquement à un référentiel neutre, tel que le sol. Il est alors possible de limiter la propagation de ces vibrations à d'autres éléments constituant le système de remplissage pouvant fausser la mesure de la quantité mesurée de poudre.

Selon un mode de réalisation du système de remplissage, l'au moins un réservoir de distribution d'une poudre pyrotechnique est associé à deux dispositifs de mesure de poudre, chaque dispositif de mesure de poudre étant associé à un dispositif d'acheminement de poudre depuis le réservoir jusqu'à une cuvette du dispositif de mesure correspondant.

Chaque dispositif de mesure du système de remplissage peut avantageusement comprendre les caractéristiques qui précédent.

Selon un aspect, le système de remplissage comprend pour chaque cuvette des dispositifs de mesure un entonnoir supérieur pour la récupération de la poudre contenue dans la cuvette correspondante.

Selon un aspect, le système de remplissage comprenant en outre un dispositif de sélection, le dispositif de sélection comportant un bac récupérateur et un entonnoir inférieur,
le dispositif de sélection est configuré pour la mise en position du bac récupérateur ou de l'entonnoir inférieur en regard de la cuvette.

Selon un aspect, l'unité de contrôle électronique est configurée pour comparer la mesure de la quantité de poudre contenue dans la cuvette à une quantité de poudre désirée.

Selon un aspect, lorsque la quantité de poudre mesurée dans la cuvette correspond à la quantité de poudre désirée, l'unité de contrôle électronique est configurée pour commander le dispositif de sélection de sorte à vérifier que l'entonnoir inférieur est disposé en regard de la cuvette.

Selon un aspect, dans ce cas, si l'entonnoir inférieur n'est pas placé en regard de la cuvette, l'unité de contrôle électronique est configurée pour commander le dispositif de sélection de sorte à placer l'entonnoir inférieur en regard de la cuvette.

Selon un aspect, lorsque la quantité de poudre mesurée dans la cuvette ne correspond pas à la quantité de poudre désirée, l'unité de contrôle électronique est configurée pour commander le dispositif de sélection de sorte à vérifier que le bac récupérateur est placé en regard de la cuvette.

Selon un aspect, dans ce cas, si le bac récupérateur n'est pas placé en regard de la cuvette, l'unité de contrôle électronique est configurée pour commander le dispositif de sélection de sorte à placer le bac récupérateur en regard de la cuvette.

Selon un mode de réalisation du système de remplissage, le système de remplissage comprend ledit entonnoir inférieur, dit premier entonnoir inférieur, et un deuxième entonnoir inférieur pour la récupération de la poudre versée dans le premier entonnoir inférieur, le deuxième entonnoir inférieur étant prévu pour s'accoupler avec le contenant de la charge pyrotechnique précédemment acheminé.

Selon un mode de réalisation du système de remplissage, une ouverture de sortie du deuxième entonnoir inférieur est biseautée pour faciliter son accouplement avec une ouverture d'entrée du contenant de la charge pyrotechnique.

Selon un aspect, lorsque la quantité de poudre mesurée dans la cuvette correspond à la quantité de poudre désirée, l'unité de contrôle électronique est configurée pour commander une ligne d'acheminement du contenant de sorte à placer le contenant en regard du premier entonnoir inférieur.

Selon un aspect, lorsque la quantité de poudre mesurée dans la cuvette correspond à la quantité de poudre désirée, l'unité de contrôle électronique est configurée pour commander une ligne d'acheminement du contenant de sorte à placer le contenant en regard du deuxième entonnoir inférieur.

Selon un aspect, lorsque la quantité de poudre mesurée dans la cuvette correspond à la quantité de poudre désirée, l'unité de contrôle électronique est configurée pour commander le deuxième entonnoir inférieur de sorte à ce qu'il s'accouple avec le contenant précédemment acheminé.

Le présent exposé concerne en outre un ensemble de remplissage comprenant :
- un premier système de remplissage conforme à l'une quelconque des caractéristiques susmentionnées ;
- un deuxième système de remplissage conforme à l'une quelconque des caractéristiques susmentionnées ;

le premier système de remplissage étant configuré pour remplir un contenant d'une charge pyrotechnique par un premier type de poudre pyrotechnique et le deuxième système de remplissage étant configuré pour remplir le contenant de la charge pyrotechnique par un deuxième type de poudre pyrotechnique.

D'autres aspects, buts et avantages de l'invention apparaîtront à la lecture de la description détaillée suivante de formes de réalisation préférées de celle-ci données à titre d'exemples non limitatifs, faite en référence aux dessins annexés suivants:
[Fig. 1] représente schématiquement un système de remplissage,
[Fig. 2] représente schématiquement le système de remplissage vue de côté,
[Fig. 3] illustre une séquence de commande en fréquence de vibration du système de distribution.

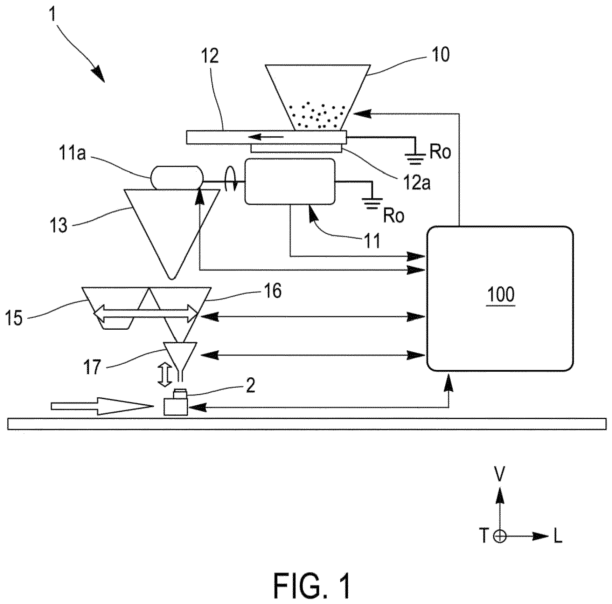

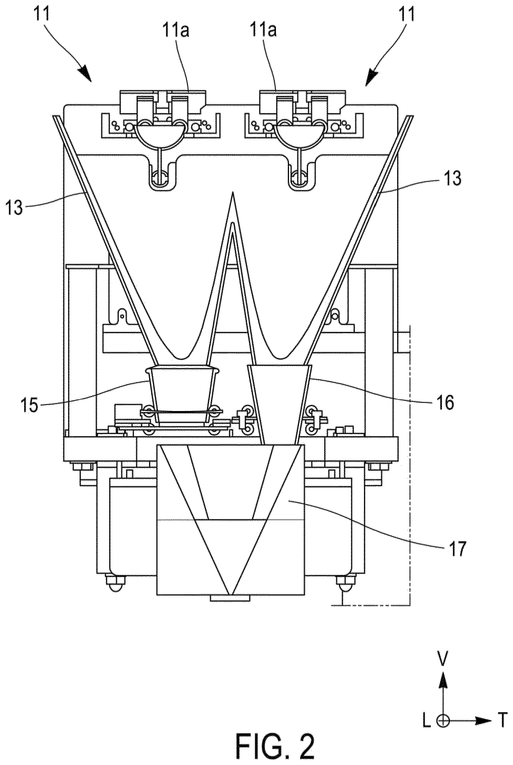

Aux figure 1 et 2, on a représenté le système de remplissage 1 d'un contenant 2 d'une charge pyrotechnique apte à mettre en oeuvre le procédé tel que défini dans le présent document.

Le système de remplissage 1 est commandé à l'aide d'une unité de contrôle électronique configurée pour réaliser les étapes du procédé décrit dans le présent document.

Le système de remplissage 1 est représenté monté sur une ligne de production pour le remplissage en série de contenant 2 de charges pyrotechniques.

A la figure 1, la direction d'avancement d'une telle ligne de production est représentée par une direction longitudinale L.

A la figure 2, la direction d'avancement est représentée par une direction orthogonal T à la direction longitudinale L.

Un direction verticale V orthogonale à chacune des directions longitudinale L et orthogonale T est également représentée.

Tel que représenté, le système de remplissage 1 comprenant au moins un réservoir de distribution 10 d'une poudre pyrotechnique, un dispositif de mesure 11 de cette poudre, et un dispositif d'acheminement 12 de cette poudre depuis le réservoir 10 jusqu'à une cuvette 11a du dispositif de mesure 11.

Un actionneur de vibrations 12a, tel qu'un actionneur piézo-électrique, est couplé au dispositif d'acheminement de cette poudre.

L'actionneur de vibrations 12a génère alors des vibrations selon une fréquence de vibration pouvant être variable.

Avantageusement, le dispositif de mesure 11 est directement relié mécaniquement à un référentiel neutre R0, tel que le sol. La dépendance mécanique à un tel référentiel permet de s'assurer que la mesure de la quantité de poudre contenue dans la cuvette 11a ne soit pas dégradée par des perturbations mécaniques des éléments constituant le système de remplissage 1.

De la même façon, l'au moins un actionneur de vibrations 12a est directement relié mécaniquement à un référentiel neutre R0, tel que le sol. Il est alors possible de limiter la propagation de ces vibrations à d'autres éléments constituant le système de remplissage 1 pouvant fausser la mesure de la quantité de poudre contenue dans la cuvette.

Le dispositif d'acheminement 12 peut avantageusement comprendre une trémie.

Le dispositif de mesure 11 peut être formé par un peson auquel est fixé la cuvette 11a formant un doseur, ici représenté en forme de cuillère.

Tel que représenté à la figure 2, le système de remplissage 1 comprend deux dispositifs de mesure 11 de poudre, chaque dispositif de mesure 11 de poudre étant associé à un dispositif d'acheminement 12 de poudre depuis le réservoir 10 jusqu'à une cuvette 11a du dispositif de mesure 11 correspondant.

Avantageusement, il peut être prévu un réservoir 10 pour chaque dispositif de mesure 11 et dispositif d'acheminement 12.

Chaque cuvette 11a des dispositifs de mesure 11 est associée à un entonnoir supérieur 13 pour la récupération de la poudre contenue dans la cuvette 11a correspondante.

Un dispositif de sélection des cuvettes 11a comporte un bac récupérateur 15 et un entonnoir inférieur 16. Le dispositif de sélection est avantageusement prévu pour positionner le bac récupérateur 15 et/ou l'entonnoir inférieur 16 en regard du ou des entonnoirs supérieurs 13.

Pour cela, le bac récupérateur 15 et/ou l'entonnoir inférieur 16 du dispositif de sélection sont guidés en translation selon la direction d'avancement de la ligne de production.

Le système de remplissage comprend ledit entonnoir inférieur, dit premier entonnoir inférieur 16, et un deuxième entonnoir inférieur 17 pour la récupération de la poudre versée dans le premier entonnoir inférieur 16, le deuxième entonnoir inférieur 17 est prévu pour s'accoupler avec le contenant 2 de la charge pyrotechnique précédemment acheminé.

Une ouverture de sortie 17a du deuxième entonnoir inférieur 17 est biseautée pour faciliter son accouplement avec une ouverture d'entrée 2a du contenant 2 de la charge pyrotechnique.

On va maintenant décrire les étapes du procédé décrit dans le présent document permettant le remplissage du contenant de la charge pyrotechnique.

Lorsque la poudre est acheminée, une étape de régulation du débit de poudre est réalisée dans laquelle chaque dispositif d'acheminement 12 est mis en vibration selon une séquence de commande en fréquence de vibration, la fréquence de vibration étant inversement proportionnelle au taux de remplissage du contenant 2 de la charge pyrotechnique.

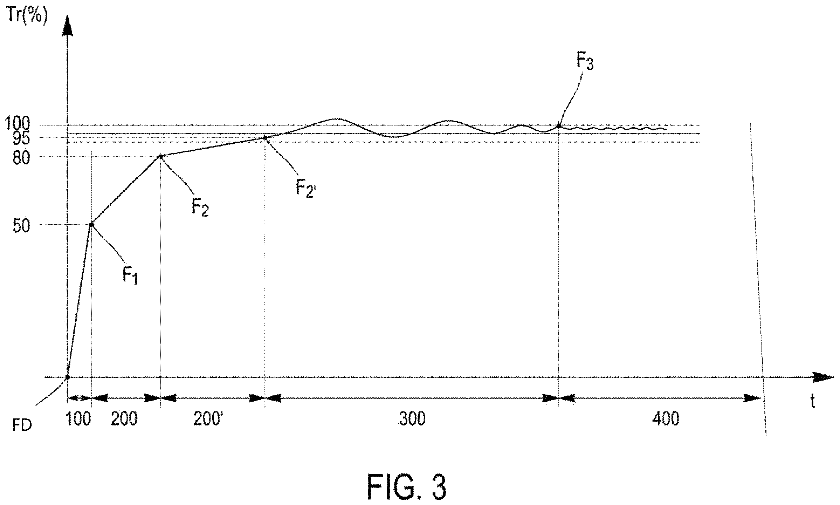

La figure 3 illustre une telle séquence de commande en fréquence de vibration représentée par le taux de remplissage Tr en pourcentage proportionnel au temps de remplissage.

La séquence de commande en fréquence de vibration comprend une première phase 100 dans laquelle le dispositif d'acheminement 12 est mis en vibration selon une première fréquence F1 comprise entre 16Hz et 18 Hz, préférentiellement 17 Hz, jusqu'à atteindre un premier taux de remplissage du contenant compris entre 50% et 80%, préférentiellement au moins égal à 75%, du taux de remplissage désiré du contenant, puis une deuxième phase 200 dans laquelle le dispositif d'acheminement 12 est mis en vibration selon une deuxième fréquence F2 comprise entre 13 Hz et 16 Hz, préférentiellement 15 Hz, jusqu'à atteindre un deuxième taux de remplissage du contenant compris entre 80% et 95 %, préférentiellement au moins égal à 90 %, du taux de remplissage désiré du contenant.

La séquence de commande en fréquence de vibration comprend, dans cet exemple, une deuxième phase analogue 200' dans laquelle le dispositif d'acheminement est mis en vibration selon une deuxième fréquence analogue F2' préférentiellement comprise entre 8 Hz et 13 Hz, préférentiellement 10 Hz, ladite deuxième fréquence analogue F2' étant inférieure à ladite deuxième fréquence F2.

La séquence de commande en fréquence de vibration comprend puis une troisième phase 300 dans laquelle la mise en vibration du dispositif d'acheminement 12 est stoppée jusqu'à la stabilisation de la quantité de poudre mesurée, par exemple pendant une durée sensiblement égale à 2000ms.

Suite à cette troisième phase, si la quantité de poudre mesurée correspond à la quantité de poudre désirée la séquence de commande en fréquence de vibration est terminée et la cuvette est considérée comme étant correctement dosée et le procédé peut être poursuivi jusqu'au remplissage du contenant de cette dose de poudre.

Si la quantité de poudre mesurée est supérieure à la quantité de poudre désirée, la séquence de commande en fréquence de vibration est terminée, la cuvette est considérée comme incorrectement dosée et le procédé peut être poursuivi jusqu'à la mise au rebut ou la récupération de cette dose de poudre.

Successivement à la troisième phase 300, si la quantité de poudre mesurée est inférieure à la quantité de poudre désirée, par exemple lorsque le taux de remplissage est compris entre 95 et 98%, il peut être réalisé une quatrième phase 400, dans laquelle le dispositif d'acheminement 12 peut avantageusement être mis en vibration selon une troisième fréquence F3 comprise entre 10 Hz et 20 Hz, et plus préférentiellement égale à 14 Hz, pendant une durée prédéterminée, par exemple égale à 1000ms, puis une nouvelle phase de stabilisation, ici cinquième phase, similaire à la troisième phase. Suite à cette cinquième phase, si la quantité de poudre mesurée correspond à la quantité de poudre désirée, la séquence de commande en fréquence de vibration est terminée et la cuvette est considérée comme étant correctement dosée et le procédé peut être poursuivi jusqu'au remplissage du contenant de cette dose de poudre.

Si la quantité de poudre mesurée est inférieure à la quantité de poudre désirée, par exemple lorsque le taux de remplissage est compris entre 95 et 98%, les quatrième et cinquième phases peuvent être répétées jusqu'à atteindre la quantité de poudre désirée.

Si la quantité de poudre mesurée est supérieure à la quantité de poudre désirée, la séquence de commande en fréquence de vibration est terminée, la cuvette est considérée comme incorrectement dosée et le procédé peut être poursuivi jusqu'à la mise au rebut ou la récupération de cette dose de poudre.

Par ailleurs, il est possible de réaliser une phase initiale où le dispositif d'acheminement 12 est mis en vibration selon une fréquence de démarrage FD pendant une durée de démarrage prédéterminée, par exemple égale à 190ms, préalablement à la première phase 100, ceci pour permettre d'atteindre plus rapidement le taux de remplissage désiré.

Par exemple, la fréquence de démarrage FD est supérieure à 18Hz. Par exemple, la fréquence de démarrage FD est comprise entre 18Hz et 20Hz. Par exemple, la fréquence de démarrage FD est sensiblement égale à 19Hz.

La première fréquence est dite « à très grande vitesse », la deuxième fréquence est dite « à grande vitesse », le deuxième fréquence analogue est dite « à petite vitesse » et la troisième fréquence et la fréquence de démarrage sont dites « boost ».

A partir de la troisième phase 300, l'acheminement des grains de poudre dans la cuvette continue par un déversement faisant suite à la dernière phase de vibration. Dans cette troisième phase 300, la mesure de la quantité de poudre contenu dans la cuvette se stabilise au cours du temps.

Parallèlement à l'étape de régulation du débit de poudre, il est réalisé une étape de mesure de la poudre contenue dans chaque cuvette 11a des dispositifs de mesure 11.

Lorsque la mesure de la poudre contenue dans au moins une cuvette 11a est égale à une valeur prédéterminée correspondant à la quantité de poudre désirée pour remplir le contenant 2 de la charge pyrotechnique, il est réalisée une étape de vérification de la position du dispositif de sélection dans laquelle il est vérifié que l'entonnoir supérieur 13 associé à ladite cuvette 11a contenant ladite valeur prédéterminée est disposé en regard de l'entonnoir inférieur 17.

Lorsqu'au moins une cuvette 11a ou bien chaque cuvette 11a est correctement dosée, l'une quelconque de ces cuvettes 11a est retenue pour verser la poudre qu'elle contient dans le contenant 2 de la charge pyrotechnique.

Lorsque la mesure de la poudre contenue dans au moins une cuvette 11a n'est pas égale à la valeur prédéterminée, il est réalisé une étape de vérification de la position du dispositif de sélection dans laquelle il est vérifié que l'entonnoir supérieur 13 associé à ladite cuvette 11a ne contenant pas ladite valeur prédéterminée est disposé en regard du bac récupérateur 15.

Lorsque le bac récupérateur 15 n'est pas en regard de l'entonnoir supérieur 13, le dispositif de sélection est déplacé pour réaliser cette mise en position.

Le contenu de la cuvette 11a incorrectement dosé est alors récupéré sans interruption de la ligne de production.

Lorsque le contenu de la cuvette 11a est correctement dosé, il est réalisé une étape de vérification de la position du dispositif de sélection dans laquelle il est vérifié que l'entonnoir supérieur 13 associé à ladite cuvette 11a correctement dosée est disposé en regard du premier entonnoir inférieur 16.

Lorsque le premier entonnoir inférieur 16 n'est pas en regard de l'entonnoir supérieur 13, le dispositif de sélection est déplacé pour réaliser cette mise en position.

Puis, il est réalisé une étape d'acheminement du contenant 2 de la charge pyrotechnique en regard du deuxième entonnoir inférieur 17, puis une étape d'accouplement du deuxième entonnoir inférieur 17 avec le contenant 2 de la charge pyrotechnique.

On comprendra qu'une cuvette 11a correctement dosée correspond à la cuvette 11a dont la mesure de la poudre contenue est égale à une valeur prédéterminée correspondant à la quantité désirée de poudre.

Dans l'étape d'accouplement, le deuxième entonnoir inférieur 17 est déplacé verticalement pour réaliser cet accouplement.

Alors le contenu en poudre de la cuvette est versé dans l'entonnoir supérieur 13 correspondant et il est acheminé par gravité jusqu'au contenant de la charge pyrotechnique précédemment acheminé. Ici, la cuvette est formée par une cuillère dont une tige est configurée pour être mise en rotation pour verser son contenu. A cet effet, le dispositif de mesure peut comprendre un moteur prévu à cet effet.

Bien entendu, les entonnoirs par lesquels sont acheminés la poudre sont disposés successivement les uns aux autres pour permettre cet acheminement.

Les entonnoirs peuvent être mis en vibration pour faciliter cet acheminement et éviter tous phénomènes d'accumulation de poudre lors de cet acheminement.

Il est également possible de prévoir de rendre équipotentiel les entonnoirs pour éviter tous phénomènes électrostatiques.

Bien évidemment, l'invention n'est pas limitée aux exemples qui viennent d'être décrits et de nombreux aménagements peuvent être apportés à ces exemples sans sortir du cadre de l'invention, qui est défini dans les revendications annexées.

Notamment, les différentes caractéristiques, formes, variantes et modes de réalisation de l'invention peuvent être associés les uns avec les autres selon diverses combinaisons dans la mesure où ils ne sont pas incompatibles ou exclusifs les uns des autres. En particulier, toutes les variantes et modes de réalisation décrits précédemment sont combinables entre eux.

## Revendications

1. Procédé de remplissage d'un contenant (2) d'une charge pyrotechnique à l'aide d'un système de remplissage (1), le système de remplissage (1) comprenant au moins :
- un réservoir de distribution (10) d'une poudre pyrotechnique,
- un dispositif de mesure (11) de cette poudre, et
- un dispositif d'acheminement (12) de cette poudre depuis le réservoir (10) jusqu'à une cuvette (11a) du dispositif de mesure (11),
ledit procédé comprenant:
- lorsque la poudre est acheminée, une étape de régulation du débit de poudre dans laquelle l'au moins un dispositif d'acheminement (12) de poudre est mis en vibration selon une séquence de commande en fréquence de vibration, la fréquence de vibration étant inversement proportionnelle au taux de remplissage du contenant (2) de la charge pyrotechnique,
ledit procédé étant **caractérisé en ce que** le système de remplissage (1) comprenant un dispositif de sélection, le dispositif de sélection comportant un bac récupérateur (15) et un entonnoir inférieur (16) configuré pour permettre le remplissage du contenant, ledit procédé comprend :
- une étape de mesure de la quantité de poudre pyrotechnique contenue dans la cuvette (11a) du dispositif de mesure (11),
- lorsque la quantité de poudre contenue dans la cuvette (11a) correspondant à une quantité de poudre désirée à introduire dans le contenant (2) de la charge pyrotechnique, une étape de vérification de la position du dispositif de sélection dans laquelle il est vérifié que la cuvette (11a) est disposée en regard de l'entonnoir inférieur (16).

2. Procédé de remplissage selon la revendication précédente, **caractérisé en ce que** la séquence de commande en fréquence de vibration comprend au moins :
- une première phase (100) dans laquelle le dispositif d'acheminement (12) de poudre est mis en vibration selon une première fréquence (F1) jusqu'à atteindre un premier taux de remplissage du contenant, puis
- une deuxième phase (200) dans laquelle le dispositif d'acheminement (12) de poudre est mis en vibration selon une deuxième fréquence (F2) jusqu'à atteindre un deuxième taux de remplissage du contenant,
la première fréquence (F1) étant supérieure à la deuxième fréquence (F2).

3. Procédé de remplissage selon la revendication 1 ou 2, **caractérisé en ce qu'**il comprend :
- lorsque la quantité de poudre contenue dans la cuvette (11a) ne correspond pas à la quantité de poudre désirée, une étape de vérification de la position du dispositif de sélection dans laquelle il est vérifié que la cuvette (11a) est disposée en regard du bac récupérateur (15).

4. Procédé de remplissage selon l'une quelconque des revendications 1 à 3, caractérisé en ce:
le système de remplissage (1) comprend un deuxième entonnoir inférieur (17) pour la récupération de la poudre versée dans l'entonnoir inférieur (16) dit « premier entonnoir inférieur »,
le procédé comprenant :
- une étape d'acheminement du contenant (2) de la charge pyrotechnique en regard du deuxième entonnoir inférieur (17),
- une étape d'accouplement du deuxième entonnoir inférieur (17) avec le contenant (2) de la charge pyrotechnique.

5. Procédé de remplissage selon l'une quelconque des revendications précédentes, **caractérisé en ce que** le système de remplissage est rendu équipotentiel pour éviter la retenue de grains de poudre par l'électrostatisme.

6. Procédé de remplissage selon l'une quelconque des revendications 1 à 5, **caractérisé en ce qu'**il comprend une étape de mise en vibration dans laquelle au moins le premier entonnoir inférieur (16) est mis en vibration constante.

7. Procédé de remplissage selon l'une quelconque des revendications précédentes, **caractérisé en ce que** l'au moins un réservoir de distribution (10) d'une poudre pyrotechnique est associé à deux dispositifs de mesure (11) de poudre, chaque dispositif de mesure (11) de poudre étant associé à un dispositif d'acheminement (12) de poudre depuis le réservoir jusqu'à une cuvette (11a) du dispositif de mesure (11) correspondant, l'étape de régulation du débit de poudre étant mise en oeuvre lorsque la poudre est acheminée par l'un et/ou l'autre des dispositifs d'acheminement.

8. Procédé de remplissage selon la revendication 7, comprenant :
- une étape de mesure de la quantité de poudre contenue dans chaque cuvette des dispositifs de mesure,
- lorsque la quantité de la poudre contenue dans au moins une cuvette (11a) correspond à la quantité de poudre désirée, une étape de vérification de la position du dispositif de sélection dans laquelle il est vérifié que la cuvette contenant la quantité de poudre correspondant à la quantité de poudre désirée est disposée en regard de l'entonnoir inférieur (16).

9. Système de remplissage (1) d'un contenant (2) d'une charge pyrotechnique, comprenant au moins :
- un réservoir de distribution (10) d'une poudre pyrotechnique,
- un dispositif de mesure (11) de cette poudre,
- un dispositif d'acheminement (12) de cette poudre depuis le réservoir (10) jusqu'à une cuvette (11a) du dispositif de mesure (11), et
un actionneur de vibrations (12a), tel qu'un actionneur piézo-électrique, couplé au dispositif d'acheminement (12) de la poudre,
**caractérisé en ce qu'**il comprend un dispositif de sélection comportant un bac récupérateur (15) et un entonnoir inférieur (16), le dispositif de sélection étant configuré pour la mise en position du bac récupérateur (15) et/ou de l'entonnoir inférieur (16) en regard de la cuvette.

10. Système de remplissage (1) selon la revendication 9, comprenant une unité de contrôle électronique configurée pour mettre en oeuvre une séquence de commande en fréquence de vibration, la fréquence de vibration étant inversement proportionnelle au taux de remplissage du contenant (2) de la charge pyrotechnique.

11. Système de remplissage (1) selon la revendication 9 ou 10, **caractérisé en ce que** le système de remplissage (1) comprend ledit entonnoir inférieur (16), dit premier entonnoir inférieur (16), et un deuxième entonnoir inférieur (17) pour la récupération de la poudre versée dans le premier entonnoir inférieur (16), **en ce que** le deuxième entonnoir inférieur (17) est prévu pour s'accoupler avec le contenant (2) d'une charge pyrotechnique précédemment acheminé.

12. Système de remplissage (1) selon l'une des revendications 9 à 11, **caractérisé en ce que** l'au moins un réservoir de distribution (10) d'une poudre pyrotechnique est associé à deux dispositifs de mesure (11) de poudre, chaque dispositif de mesure (11) de poudre étant associé à un dispositif d'acheminement (12) de poudre depuis le réservoir jusqu'à une cuvette (11a) du dispositif de mesure (11) correspondant.

13. Ensemble de remplissage comprenant :
- un premier système de remplissage conforme à l'une quelconque des revendications 9 à 12 ;
- un deuxième système de remplissage conforme à l'une quelconque des revendications 9 à 12;
le premier système de remplissage étant configuré pour remplir un contenant d'une charge pyrotechnique par un premier type de poudre pyrotechnique et le deuxième système de remplissage étant configuré pour remplir le contenant de la charge pyrotechnique par un deuxième type de poudre pyrotechnique.

## Patentansprüche

1. Verfahren zum Füllen eines Behälters (2) mit einer pyrotechnischen Ladung mit Hilfe eines Füllsystems (1), wobei das Füllsystem (1) mindestens Folgendes umfasst:
- einen Tank zum Verteilen (10) eines pyrotechnischen Pulvers,
- eine Vorrichtung zum Messen (11) des Pulvers, und
- eine Vorrichtung zum Befördern (12) des Pulvers aus dem Tank (10) bis in eine Küvette (11a) der Messvorrichtung (11),
wobei das Verfahren Folgendes umfasst:
- wenn das Pulver befördert wird, einen Schritt des Regulierens der Pulverdurchflussrate, wobei die mindestens eine Pulverbeförderungsvorrichtung (12) gemäß einer Schwingungsfrequenz-Steuersequenz in Schwingung versetzt wird, wobei die Schwingungsfrequenz umgekehrt proportional zum Füllgrad des Behälters (2) für die pyrotechnische Ladung ist,
wobei das Verfahren **dadurch gekennzeichnet ist, dass** das Füllsystem (1) eine Auswahlvorrichtung umfasst, wobei die Auswahlvorrichtung einen Sammelbehälter (15) und einen unteren Trichter (16) umfasst, der dazu konfiguriert ist, das Befüllen des Behälters zu ermöglichen, wobei das Verfahren Folgendes umfasst:
- einen Schritt des Messens der Menge an pyrotechnischem Pulver, die in der Küvette (11a) der Messvorrichtung (11) enthalten ist,
- wenn die in der Küvette (11a) enthaltene Pulvermenge einer gewünschten Pulvermenge entspricht, die in den Behälter (2) für die pyrotechnische Ladung eingebracht werden soll, einen Schritt des Überprüfens der Position der Auswahlvorrichtung, bei dem überprüft wird, ob die Küvette (11a) dem unteren Trichter (16) gegenüberliegend angeordnet ist.

2. Füllverfahren nach dem vorhergehenden Anspruch, **dadurch gekennzeichnet, dass** die Schwingungsfrequenz-Steuersequenz mindestens Folgendes umfasst:
- eine erste Phase (100), in der die Pulverbeförderungsvorrichtung (12) gemäß einer ersten Frequenz (F1) in Schwingung versetzt wird, bis ein erster Füllgrad des Behälters erreicht ist, dann
- eine zweite Phase (200), in der die Pulverbeförderungsvorrichtung (12) gemäß einer zweiten Frequenz (F2) in Schwingung versetzt wird, bis ein zweiter Füllgrad des Behälters erreicht ist,
wobei die erste Frequenz (F1) höher ist als die zweite Frequenz (F2).

3. Füllverfahren nach Anspruch 1 oder 2, **dadurch gekennzeichnet, dass** es Folgendes umfasst:
- wenn die in der Küvette (11a) enthaltene Pulvermenge nicht der gewünschten Pulvermenge entspricht, einen Schritt des Überprüfens der Position der Auswahlvorrichtung, bei dem überprüft wird, ob die Küvette (11a) dem Sammelbehälter (15) gegenüberliegend angeordnet ist.

4. Füllverfahren nach einem der Ansprüche 1 bis 3, **dadurch gekennzeichnet, dass**:
das Füllsystem (1) einen zweiten unteren Trichter (17) zum Sammeln des in den unteren Trichter (16), den sogenannten ersten unteren Trichter, eingefüllten Pulvers umfasst,
wobei das Verfahren Folgendes umfasst:
- einen Schritt des Beförderns des Behälters (2) für die pyrotechnische Ladung gegenüber des zweiten unteren Trichters (17),
- einen Schritt des Koppelns des zweiten unteren Trichters (17) mit dem Behälter (2) der pyrotechnischen Ladung.

5. Füllverfahren nach einem der vorhergehenden Ansprüche, **dadurch gekennzeichnet, dass** das Füllsystem äquipotential gemacht wird, um das Zurückhalten von Pulverkörnern durch Elektrostatik zu verhindern.

6. Füllverfahren nach einem der Ansprüche 1 bis 5, **dadurch gekennzeichnet, dass** es einen Schritt des Versetzens in Schwingung umfasst, bei dem mindestens der erste untere Trichter (16) in konstante Schwingung versetzt wird.

7. Füllverfahren nach einem der vorhergehenden Ansprüche, **dadurch gekennzeichnet, dass** der mindestens eine Verteilungstank (10) für ein pyrotechnisches Pulver zwei Messvorrichtungen (11) zugeordnet sind, wobei jede Pulvermessvorrichtung (11) einer Pulverbeförderungsvorrichtung (12) von dem Tank aus bis zu einer Küvette (11a) der entsprechenden Messvorrichtung (11) zugeordnet ist, wobei der Schritt des Regulierens der Pulverdurchflussrate durchgeführt wird, wenn das Pulver von der einen und/oder der anderen der Beförderungsvorrichtungen befördert wird.

8. Füllverfahren nach Anspruch 7, das Folgendes umfasst:
- einen Schritt des Messens der Pulvermenge, die in jeder Küvette der Messvorrichtungen enthalten ist,
- wenn die Menge des in mindestens einer Küvette (11a) enthaltenen Pulvers der gewünschten Pulvermenge entspricht, einen Schritt des Überprüfens der Position der Auswahlvorrichtung, bei dem überprüft wird, ob die Küvette, die die der gewünschten Pulvermenge entsprechende Pulvermenge enthält, dem ersten unteren Trichter (16) zugewandt angeordnet ist.

9. System zum Füllen (1) eines Behälters (2) mit einer pyrotechnischen Ladung, das mindestens Folgendes umfasst:
- einen Tank zum Verteilen (10) eines pyrotechnischen Pulvers,
- eine Vorrichtung zum Messen (11) des Pulvers,
- eine Vorrichtung zum Befördern (12) des Pulvers aus dem Tank (10) bis in eine Küvette (11a) der Messvorrichtung (11), und
einen Schwingungsaktuator (12a), wie etwa einen piezoelektrischen Aktuator, der mit der Pulverbeförderungsvorrichtung (12) gekoppelt ist,
**dadurch gekennzeichnet, dass** es eine Auswahlvorrichtung umfasst, die einen Sammelbehälter (15) und einen unteren Trichter (16) umfasst, wobei die Auswahlvorrichtung dazu konfiguriert ist, den Sammelbehälter (15) und/oder den unteren Trichter (16) gegenüber der Küvette zu platzieren.

10. Füllsystem (1) nach Anspruch 9, das eine elektronische Steuereinheit umfasst, die dazu konfiguriert ist, eine Schwingungsfrequenz-Steuersequenz zu implementieren, wobei die Schwingungsfrequenz umgekehrt proportional zum Füllgrad des Behälters (2) der pyrotechnischen Ladung ist.

11. Füllsystem (1) nach Anspruch 9 oder 10, **dadurch gekennzeichnet, dass** das Füllsystem (1) den unteren Trichter (16), den sogenannten ersten unteren Trichter (16), und einen zweiten unteren Trichter (17) zum Sammeln des in den ersten unteren Trichter (16) eingefüllten Pulvers umfasst, dass der zweite untere Trichter (17) so vorgesehen ist, dass er sich mit dem Behälter (2) mit einer zuvor beförderten pyrotechnischen Ladung koppelt.

12. Füllsystem (1) nach einem der Ansprüche 9 bis 11, **dadurch gekennzeichnet, dass** der mindestens eine Verteilungstank (10) für ein pyrotechnisches Pulver zwei Pulvermessvorrichtungen (11) zugeordnet sind, wobei jede Pulvermessvorrichtung (11) einer Pulverbeförderungsvorrichtung (12) von dem Tank aus bis zu einer Küvette (11a) der entsprechenden Messvorrichtung (11) zugeordnet ist.

13. Füllanordnung, die Folgendes umfasst:
- ein erstes Füllsystem nach einem der Ansprüche 9 bis 12;
- ein zweites Füllsystem nach einem der Ansprüche 9 bis 12;
wobei das erste Füllsystem dazu konfiguriert ist, einen Behälter für die pyrotechnische Ladung mit einer ersten Art von pyrotechnischem Pulver zu befüllen, und das zweite Füllsystem dazu konfiguriert ist, den Behälter für die pyrotechnische Ladung mit einer zweiten Art von pyrotechnischem Pulver zu befüllen.

## Claims

1. A method for filling a container (2) with a pyrotechnic charge using a filling system (1), the filling system (1) comprising at least:
- one tank (10) for distributing a pyrotechnic powder,
- one device (11) for measuring this powder, and
- one device (12) for conveying this powder from the tank (10) to a bowl (11a) of the measuring device (11),
said method comprising:
- when the powder is conveyed, a powder flow rate regulation step in which the at least one powder conveying device (12) is vibrated according to a vibration frequency control sequence, the vibration frequency being inversely proportional to the fill rate of the container (2) of the pyrotechnic charge,
said method being **characterized in that** the filling system (1) comprising a selection device, the selection device including a collector tray (15) and a lower funnel (16) configured to allow filling the container, said method comprises:
- a step of measuring the quantity of pyrotechnic powder contained in the bowl (11a) of the measuring device (11),
- when the quantity of powder contained in the bowl (11a) corresponding to a desired powder quantity to be introduced into the container (2) of the pyrotechnic charge, a step of checking the position of the selection device in which it is checked that the bowl (11a) is arranged opposite the lower funnel (16).

2. The filling method according to the preceding claim, **characterized in that** the vibration frequency control sequence comprises at least:
- one first phase (100) in which the powder conveying device (12) is vibrated according to a first frequency (F1) until a first container fill rate is reached, then
- one second phase (200) in which the powder conveying device (12) is vibrated according to a second frequency (F2) until a second container fill rate is reached,
the first frequency (F1) being greater than the second frequency (F2).

3. The filling method according to claim 1 or 2, **characterized in that** it comprises:
- when the quantity of powder contained in the bowl (11a) does not correspond to the desired powder quantity, a step of checking the position of the selection device in which it is checked that the bowl (11a) is arranged opposite the collector tray (15).

4. The filling method according to any one of claims 1 to 3, **characterized in that**:
the filling system (1) comprises a second lower funnel (17) for collecting the powder poured into the lower funnel (16) called the « first lower funnel »,
the method comprising:
- a step of conveying the container (2) of the pyrotechnic charge opposite the second lower funnel (17),
- a step of coupling the second lower funnel (17) with the container (2) of the pyrotechnic charge.

5. The filling method according to any one of the preceding claims, **characterized in that** the filling system is made equipotential to avoid retaining the powder grains by electrostatics.

6. The filling method according to any one of claims 1 to 5, **characterized in that** it comprises a vibrating step in which at least the first lower funnel (16) is constantly vibrated.

7. The filling method according to any one of the preceding claims, **characterized in that** the at least one tank (10) for distributing a pyrotechnic powder is associated with two powder measuring devices (11), each powder measuring device (11) being associated with a device (12) for conveying the powder from the tank to a bowl (11a) of the corresponding measuring device (11), the powder flow rate regulating step being implemented when the powder is conveyed by one or both of the conveying devices.

8. The filling method according to claim 7, comprising:
- a step of measuring the quantity of powder contained in each bowl of the measuring devices,
- when the quantity of powder contained in at least one bowl (11a) corresponds to the desired powder quantity, a step of checking the position of the selection device in which it is checked that the bowl containing the powder quantity corresponding to the desired powder quantity is arranged opposite the lower funnel (16).

9. A system (1) for filling a container (2) with a pyrotechnic charge, comprising at least:
- one tank (10) for distributing a pyrotechnic powder,
- one device (11) for measuring this powder,
- one device (12) for conveying this powder from the tank (10) to a bowl (11a) of the measuring device (11), and
a vibration actuator (12a), such as a piezoelectric actuator, coupled to the powder conveying device (12),
**characterized in that** it comprises a selection device including a collector tray (15) and a lower funnel (16), the selection device being configured to position the collector tray (15) and/or the lower funnel (16) opposite the bowl.

10. The filling system (1) according to claim 9, comprising an electronic control unit configured to implement a vibration frequency control sequence, the vibration frequency being inversely proportional to the fill rate of the container (2) of the pyrotechnic charge.

11. The filling system (1) according to claim 9 or 10, **characterized in that** the filling system (1) comprises said lower funnel (16), called the first lower funnel (16), and a second lower funnel (17) for collecting the powder poured into the first lower funnel (16), **in that** the second lower funnel (17) is provided to be coupled with the container (2) of a previously conveyed pyrotechnic charge.

12. The filling system (1) according to any of claims 9 to 11, **characterized in that** the at least one tank (10) for distributing a pyrotechnic powder is associated with two powder measuring devices (11), each powder measuring device (11) being associated with a device (12) for conveying the powder from the tank to a bowl (11a) of the corresponding measuring device (11).

13. A filling assembly comprising
- a first filling system according to any one of claims 9 to 12;
- a second filling system according to any one of claims 9 to 12;
the first filling system being configured to fill a container of a pyrotechnic charge with a first type of pyrotechnic powder and the second filling system being configured to fill the container of the pyrotechnic charge with a second type of pyrotechnic powder.
